(19) Europäisches Patentamt
European Patent Office
Office européen des brevets

(11) Veröffentlichungsnummer: **0 064 303**
**B1**

(12) **EUROPÄISCHE PATENTSCHRIFT**

(45) Veröffentlichungstag der Patentschrift: **10.04.85**

(51) Int. Cl.⁴: **C 07 C 121/60,** D 06 L 3/12

(21) Anmeldenummer: **82104463.3**

(22) Anmeldetag: **31.12.80**

(60) Veröffentlichungsnummer der früheren Anmeldung nach Art. 76 EPÜ:

(54) **Gemische von Biscyanstyrylbenzolen.**

(30) Priorität: **12.01.80 DE 3001065**

(43) Veröffentlichungstag der Anmeldung: **10.11.82 Patentblatt 82/45**

(45) Bekanntmachung des Hinweises auf die Patenterteilung: **10.04.85 Patentblatt 85/15**

(84) Benannte Vertragsstaaten: **CH DE FR GB IT LI**

(56) Entgegenhaltungen:
**EP - A - 0 023 027**
**EP - A - 0 030 917**
**CH - A - 416 078**
**FR - A - 1 415 977**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Seybold,Guenther, Dr., Friedrich-Ebert-Strasse 4, D-6708 Neuhofen (DE)**

**EP 0 064 303 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Verbindungsgemische, die Verbindungen der Formel

-CH=CH- -CH=CH-
CN CN

im ungefähren Verhältnis

a) o,p' : o,o' = 65 : 35
oder
b) o,p' : o,o' : o,m' = 27 : 13 : 60
oder
c) o,p' : o,m' = 60 : 40

enthalten.

Die erfindungsgemässen Gemische eignen sich hervorragend zum optischen Aufhellen von insbesondere synthetischen Polyestern.

Die Herstellung der erfindungsgemässen Mischungen kann den Beispielen entnommen werden, in denen sich Angaben über Teile und Prozente, sofern nicht anders vermerkt, auf das Gewicht beziehen.

Gegenüber einem in Beispiel 7 der US-PS Nr. 3 294 570 beschriebenen Gemisch zeichnen sich die erfindungsgemässen Mischungen durch eine starke Erniedrigung der Fixiertemperatur beim Thermosolverfahren, Verbesserung des Aufziehverhaltens beim HT-Verfahren sowie durch eine wesentlich gesteigerte Ausgiebigkeit aus.

Weiterhin ist überraschenderweise die Eigenfarbe erfindungsgemässer Mischungen wesentlich geringer, wodurch zusammen mit der besseren Fixierung, die z.B. bei den bekannten Aufhellern der Biscyanstyrylbenzolreihe auftretende Kantenmarkierung der Stoffbahnen im Spannrahmen nicht mehr auftritt.

In der Regel erhält man mit den erfindungsgemässen Mischungen auch noch einen höheren Weissgrad im Vergleich zu bekannten Aufhellern ähnlicher Struktur.

*Beispiel 1*

Zu einer Mischung aus 10 Teilen Methylglykolacetat, 1,4 Teilen Terephthalaldehyd und 3,3 Teilen o-Cyanbenzylphosphonsäurediethylester wurden bei 120 U/min und 28 bis 32°C innerhalb von 5 Stunden 2,34 Teile einer 30%igen Natriummethylatlösung zugetropft. Nach 4stündigem Nachrühren bei 40 bis 45°C wurden 1,9 Teile p-Cyanbenzylphosphonester zugegeben und anschliessend innerhalb von 6 Stunden bei 35 bis 40°C 1,7 Teile einer 30%igen Lösung von Natriummethanolat in Methanol zugetropft. Nach 6stündigem Nachrühren bei 45 bis 50°C wurde der grünlich weisse Niederschlag abgesaugt.

Ausbeute: 2,55 Teile

Laut gaschromatographischer Analyse handelt es sich um ein Gemisch von

65% NC- -CH=CH- -CH=CH-
CN

und

35% -CH=CH- -CH=CH-
CN CN

Dieses Gemisch zeigte als Aufheller für PES besonders günstige Eigenschaften. Hervorzuheben sind das für Bisstyrylderivate ausserordentlich günstige Fixiervermögen und die hohe Ausgiebigkeit.

*Beispiel 2*

Zu einer Mischung von 100 Teilen Methylglykolacetat, 14,4 Teilen Terephthalaldehyd und 28,6 Teilen o-Cyanbenzylphosphorsäurediethylester werden bei 120 U/min und 28 bis 32°C innerhalb von 5 Stunden 20,3 Teile einer 30%igen Natriummethylatlösung zugetropft. Nach 4stündigem Nachrühren bei 40 bis 45°C werden 15,2 Teile m-Cyanbenzylphosphonester, gefolgt von 10,8 Teilen einer 30%igen Natriummethylatlösung zugegeben und erneut wird 1 Stunde bei 35°C gerührt. Anschliessend gibt man 6,8 Teile p-Cyanbenzylphosphonsäurediethylester und 5 Teile 30%ige Natriummethylatlösung zu und rührt 5 Stunden bei 35 bis 40°C nach.

Nach Aufarbeitung wie in Beispiel 1 beschrieben erhält man ein grünlich weisses kristallines Produkt, das laut gaschromatographischer Analyse

60% -CH=CH- -CH=CH-
CN NC

27% NC- -CH=CH- -CH=CH-
NC

und

13% -CH=CH- -CH=CH-
CN NC

enthält. Dieses Gemisch hellt Polyestergewebe hervorragend auf. Hervorzuheben sind die günstigen Fixiereigenschaften, die rote Nuance und die Ausgiebigkeit.

*Beispiel 3*

Man verfährt wie in Beispiel 2 und setzt 14,4 Teile Terephthalaldehyd nacheinander mit 25 Teilen o-Cyanbenzylphosphonester, 15,2 Teilen m-Cyanbenzylphosphonester und 10 Teilen p-Cyanbenzylphosphonester um. Man erhält ein Aufhellungsgemisch von

60% -CH=CH- -CH=CH-
CN CN

und

40% (2-CN-C6H4)-CH=CH-(C6H4)-CH=CH-(C6H4)-CN,

das ebenfalls hervorragend zur Polyesteraufhellung geeignet ist.

**Patentansprüche**

1. Gemische von Biscyanstyrylbenzolen, die Verbindungen der Formel

(2-CN-C6H4)-CH=CH-(C6H4)-CH=CH-(C6H4)-CN

im ungefähren Verhältnis

a) o,p' : o,o' = 65 : 35
 oder
b) o,p' : o,o' : o,m' = 27 : 13 : 60
 oder
c) o,p' : o,m' = 60 : 40

enthalten.

2. Verwendung der Gemische gemäss Anspruch 1 zum optischen Aufhellen von synthetischen Polyestern.

**Claims**

1. Mixtures of dicyanostyrylbenzenes, which contain compounds of the formula

(2-CN-C6H4)-CH=CH-(C6H4)-CH=CH-(C6H4)-CN

the ratio of

a) o,p'- to o,o'-compound being about 65 : 35,
b) o,p'- to o,o'- to o,m'-compound about 27 : 13 : 60, or
c) o,p'- to o,m'-compound about 60 : 40.

2. The use of the mixtures as claimed in claim 1 for the optical brightening of synthetic polyesters.

**Revendications**

1. Mélanges de biscyanostyrylbenzènes qui contiennent des composés de formule

(2-CN-C6H4)-CH=CH-(C6H4)-CH=CH-(C6H4)-CN

dans le rapport approximatif

a) o,p' : o,o' = 65 : 35
 ou
b) o,p' : o,o' : o,m' = 27 : 13 : 60
 ou
c) o,p' : o,m' = 60 : 40.

2. Utilisation des mélanges selon la revendication 1 pour l'éclaircissage optique de polyesters synthétiques.